# EUROPEAN PATENT SPECIFICATION

(11) **EP 1 339 666 B1**
(45) Date of publication and mention of the grant of the patent: **02.04.2008**
(21) Application number: 01976475.2
(22) Date of filing: 18.10.2001
(51) Int. Cl.: C07C 209/48, C07C 211/29, C07C 29/09, C07C 33/46

(54) **PREPARATION OF 4-METHYL-2,3,5,6-TETRAFLUOROBENZYL ALCOHOL**
HERSTELLUNG VON 4-METHYL-2,3,5,6-TETRAFLUORBENZYLALKOHOL
PREPARATION D'ALCOOL 4-METHYL-2,3,5,6-TETRAFLUOROBENZYLIQUE

(30) Priority: 27.10.2000 GB 0026349
(43) Date of publication of application: 03.09.2003
(73) Proprietor: Syngenta Limited, Guildford Surrey GU2 7YH (GB)
(72) Inventor: JONES, Raymond Vincent, Grangemouth Stirlingshire FK3 8XG (GB)
(74) Representative: Waterman, John Richard
(86) International application number: PCT/GB2001/004648
(87) International publication number: WO 2002/034706

(56) References cited:
- EP-A- 0 099 622
- WO-A-01/55064
- US-A- 4 405 640
- SUMITOMO CHEMICAL CO ET AL: "Hydroxydiphenylamines" CHEMABS, XP002169098

## Description

The present invention relates to a process for making polyfluorinated benzyl alcohols which are useful in the synthesis of pyrethroid pesticides.

Esters of *cis*-3-(haloalkenyl)-2,2-dimethylcyclopropanecarboxylic acid with 4-methyl-2,3,5,6-tetrafluorobenzyl alcohol, in particular tefluthrin [2,3,5,6-tetrafluoro-4-methylbenzyl *cis*-3-((Z)-2-chloro-3,3,3-trifluoroprop-1-enyl)-2,2-dimethylcyclopropane carboxylate] are important insecticidal and acaricidal products. It is thus desirable to have an industrially acceptable and efficient process for making the necessary intermediates such as 4-methyl-2,3,5,6-tetrafluorobenzyl alcohol.

Known processes for making of 4-methyl-2,3,5,6-tetrafluorobenzyl alcohol are described in DE3714602, GB2155464 and EP31199 but the processes involve reduction of the parent benzoic acids or acid halides using expensive reagents such as borohydrides, or lithiation of tetrafluorobenzene at very low temperature followed by methylation. These methods are not suited to industrial application as they involve the use of expensive reagents or require specialised low temperature conditions.

The applicants have found that these disadvantages may be avoided by preparing 4-methyl-2,3,5,6-tetrafluorobenzyl alcohol from 1,4-bis(hydroxymethyl)-2,3,5,6-tetrafluorobenzene and have devised a practical and efficient process for the manufacture of both compounds on an industrial scale starting from the readily available compound 2,3,5,6-tetrachloroterephthalonitrile.

There is therefore provided a method for producing 1,4-bis(hydroxymethyl)-2,3,5,6-tetrafluorobenzene comprising:
a) fluorinating 2,3,5,6-tetrachloroterephthalonitrile to obtain 2,3,5,6-tetrafluoroterephthalonitrile;
b) hydrogenating 2,3,5,6-tetrafluoroterephthalonitrile to give 1,4-bis(aminomethyl)-2,3,5,6-tetrafluorobenzene; and
c) converting 1,4-bis(aminomethyl)-2,3,5,6-tetrafluorobenzene to 1,4-bis(hydroxymethyl)-2,3,5,6-tetrafluorobenzene.

The fluorination of step a) is suitably carried out using fluoride exchange agents such as alkali metal fluorides, for example potassium fluoride and optionally a phase transfer catalyst, for example tetramethyl ammonium chloride (TMAC). The reaction is suitably carried out in a solvent. Suitable solvents are dipolar aprotic solvents such as dimethylfomamide. The reaction is suitably performed at temperatures between 100-150°C, preferably at 130-150°C and preferably under anhydrous conditions.

The hydrogenation process of step b) may be carried out according to any of the procedures in described in EP99622. A particularly suitable method employs hydrogen and a metal catalyst such as palladium. The reaction may be carried out at from 0°C to about 60°C and from ambient pressure to 10bar of over pressure. Suitable solvents for use in the reaction include alcohols, carboxylic acids, such as formic acid, acetic acid, or mixtures of either class with water. A preferred solvent for the hydrogenation reaction is acetic acid or aqueous acetic acid. The mol ratio of acetic acid is suitably 2-10:1 but is preferably 4-6:1

Step c) is preferably performed by diazotisation and *in-situ* hydrolytic decomposition of the diazonium salt. The diazotisation process is suitably performed using aqueous sodium nitrite and an acid at a temperature of 0-100°C , preferably 50-80°C. The concentration of the amine in the reaction (prior to nitrite addition) is suitably 5 - 30%, preferably 5-15%. The mol ratio of nitrite to 1,4-bis(aminomethyl)-2,3,5,6-tetrafluorobenzene is suitably 2-5:1 but 2:1 is preferred. After completion of step c), the product may contain some esters (e.g. acetate esters) of the desired benzyl alcohol product. The acetate ester structures are shown below. 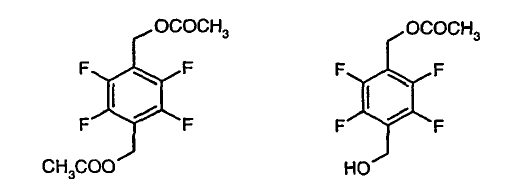 It has been found advantageous to render the reaction mass alkaline and then heat it to hydrolyse the esters to the desired diol compound. Once the esters have all been hydrolysed, the reaction mass is adjusted to between pH 7-10, preferably to between pH 9-10 prior to extraction of the product into a solvent for purification or for use in subsequent processes.

In a further aspect of the invention the 1,4-bis(hydroxymethyl)-2,3,5,6-tetrafluorobenzene from step c) may be converted to 4-methyl-2,3,5,6-tetrafluorobenzyl alcohol by
i) halogenation of 1,4-bis(hydroxymethyl)-2,3,5,6-tetrafluorobenzene to give a 2,3,5,6-tetrafluoro-4-(halomethyl)benzyl alcohol; and
ii) hydrogenation of the 2,3,5,6-tetrafluoro-4-(halomethyl)benzyl alcohol to give 4-methyl-2,3,5,6-tetrafluorobenzyl alcohol.

The halogenation of step i) where the halo group is for example chloro or bromo is suitably carried out using halogenating agents such as aqueous hydrochloric acid or hydrobromic acid. The reaction is suitably carried out in two phases in the presence of a solvent. Suitable solvents are inert and water immiscible solvents, such as aromatic hydrocarbons, for example toluene. The reaction is performed at a temperature between 50-150°C, preferably at 75-100°C.

The hydrogenation reaction of step ii) may be performed under process conditions similar to those for outlined for step b) above but in the presence of a base to absorb the liberated hydrogen halide. Suitable bases are alkali or alkaline earth metal hydroxides or carbonates or alkaline earth metal oxides. Suitable solvents are alcohols, esters or aromatic hydrocarbons.

In yet a further aspect of the invention, the 4-methyl-2,3,5,6-tetrafluorobenzyl alcohol is reacted with cis-Z 3-(2-chloro-1,1,1-trifluoro-2-propenyl)-2,2-dimethylcyclopropane carbonyl chloride 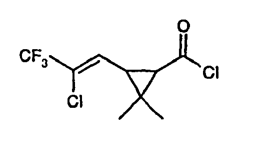 to give tefluthrin.

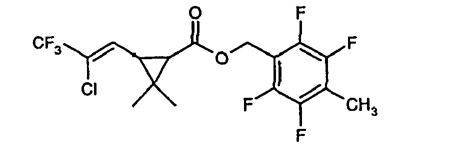

Suitable conditions for performing the reaction are described for example in EP-A-31199.

In one preferred embodiment of the invention, the 2,3,5,6-tetrafluoroterephthalonitrile is hydrogenated in a carboxylic acid solvent, for example acetic acid, and the hydrogenation mass is passed straight into the diazotisation stage (step c)) after screening, and no further acid is then required.

In another preferred embodiment 1,4-bis(hydroxymethyl)-2,3,5,6-tetrafluorobenzene from step c) is extracted from the reaction mass in a solvent such as methyl isobutylketone (MiBK), the solvent removed under reduced pressure and a second solvent is added, the second solvent being suitable for the halogenation reaction of step i). A suitable second solvent is toluene.

In a further preferred embodiment, the 1,4-bis(hydroxymethyl)-2,3,5,6-tetrafluorobenzene from step c) is extracted directly into a solvent that is also used in the halogenation reaction of step i). Suitable solvents are those that are stable to aqueous acid, such as aromatic hydrocarbons (for example toluene, xylene or a halobenzene) or halogenated hydrocarbons (for example ethylene dichloride or perchloroethylene).

The following Examples illustrate the processes and the compounds of this invention.

1H NMR was carried out on a Bruker AS 200 (200 MHz 1H) spectrometer, using TMS as reference standard. Gas chromatography analyses were obtained with a Hewlett Packard 5890 Series II. Hydrogenation reactions under pressure were carried out in a 1 litre stainless steel autoclave, fitted with a gas-inducing turbine agitator at 1000 rpm. Hydrogen feed was through a dip-pipe via a Buchi gas controller type 6002, heating and cooling was controlled by a Jelabo FP40 bath. Palladium on carbon type 58 catalyst was supplied by Johnson Matthey Ltd.

### EXAMPLE 1

This Example describes the preparation of 1,4-bis(hydroxymethyl)-2,3,5,6-tetrafluorobenzene

### Step a. Preparation of 2,3,5,6-tetrafluoroterephthalonitrile

Potassium fluoride (117.7 g) and DMF (330 g) were charged to a 1 litre jacketed flask fitted with a condenser, nitrogen blanket and external heating cooling coil. The mixture was agitated under a nitrogen atmosphere for 5 minutes after which time a sample (5 ml) was taken and analysed for water content to ensure that the reaction mixture contained <0.2 wt% water. 2,3,5,6-Tetrachloroterephthalonitrile (120 g) and tetramethylammonium chloride (1.55 g) were then charged and the reaction mixture heated to 115°C under agitation. The reaction was maintained at 115°C under nitrogen for 6 hours after which time a sample was taken to determine if completion of reaction had been achieved (i.e. monochloro impurity was < 2%). The reaction mixture was further heated at 115°C for 30 minutes if necessary. The reaction was cooled to 70°C and dropped out of the reaction vessel via the bottom run off valve into a stirred 2 litre beaker containing water (670 g) at ambient temperature. The resulting slurry was filtered and the filter cake water washed (3 x 300 ml). The 2,3,5,6-tetrafluoroterephthalonitrile was partially dried on the filter and discharged into a suitable storage vessel. The reaction produced 97.8 g of water wet paste, GC analysis showed a strength of 78.6 % giving a yield of 85.2 %.

### Step b. Preparation of 1,4-bis(aminomethyl)-2,3,5,6-tetrafluorobenzene

Water (258 g), acetic acid (179 g), 2,3,5,6-tetrafluoroterephthalonitrile paste (90.6 g 100% wt.), wetting agent (Nopco 8050) (0.15 g) and 5% palladium/carbon catalyst, (1.8 g 100% wt.) were charged to the autoclave. The lid was sealed and the autoclave was initially purged with nitrogen to remove residual oxygen and then pressurised to 5 bar g. with hydrogen after which the gas controller was zeroed. The agitator was started and the pressure maintained at 5 bar g. automatically by the Buchi controller. The temperature was monitored manually and the reaction exotherm controlled by manual intervention using the external heating / cooling bath. The reaction temperature was initially at 5°C rising to 30°C over the first 120 minutes of hydrogen ingress. The hydrogen consumption was totalled by the Buchi gas controller. The reaction was continued until the consumption of hydrogen ceased (typically 4 hrs.). The residual pressure was released and the autoclave purged with nitrogen. The contents of the autoclave were discharged by the autoclave's bottom run-off valve, followed by a small water wash. The spent catalyst was removed by filtration. The filtrates and filter-cake water washes were combined and analysed for product 1,4-bis(aminomethyl)-2,3,5,6-tetrafluorobenzene. The reaction produced 83.4 g of diamine, this equating to a reaction yield of 88.6%.

### Step c. Preparation of 1,4-bis(hydroxymethyl)-2,3,5,6-tetrafluorobenzene

1,4-Bis(aminomethyl)-2,3,5,6-tetrafluorobenzene (68.6 g), acetic acid (104 g), water (451 g) and a silicone-based defoaming agent (0.2 g) were charged to a 1 litre split neck reaction flask, agitation was maintained throughout. The flask was held at 25 °C and aqueous sodium nitrite solution (36%, 134.2 g) was added over 90 minutes. The reaction temperature was increased to 70 °C over the course of the addition. The reaction was held at 70 °C for a further 30 minutes following completion of the nitrite addition. The reaction mass was sampled and analysed for residual sodium nitrite using starch-iodide paper. Upon confirmation that excess sodium nitrite had been charged (starch-iodide paper turns blue) the reaction was cooled to 60 °C. The by-product acetate esters of the product were next hydrolysed by treatment with sodium hydroxide. Sufficient 47% aqueous sodium hydroxide solution was charged to raise the pH to >11.5. The temperature was then raised to 90 °C and held for 30 minutes after which time the pH was re-checked to ensure it was > 11.5. If pH was not >11.5, further sodium hydroxide solution was added and agitation maintained for a further 30 minutes. Reaction temperature was lowered to 60 °C and the pH adjusted to 9 by addition of hydrochloric acid. This solution was then extracted with 4-methylpentan-2-one (MiBK, 2 x 250 ml), the organic extracts were combined and analysed to show a 90% yield of 1,4-bis(hydroxymethyl)-2,3,5,6-tetrafluorobenzene by quantitative GLC analysis.

### EXAMPLE 2

This Example describes the preparation of 4-methyl-2,3,5,6-tetrafluoro-benzyl alcohol

### Step d. Preparation of 2,3,5,6-tetrafluoro-4-(bromomethyl)benzyl alcohol

A solution of 80gms 1,4-bis(hydroxymethyl)-2,3,5,6-tetrafluorobenzene in MiBK produced as in Example 1 was charged to a 1 litre reaction flask fitted for distillation (both reflux return and total take-off). The solvent was removed by distillation and then steam distillation. Toluene (303g) was added and residual water removed by azeotropic distillation. The solution was agitated and heated to 60 °C for 30 minutes and Silcolapse (0.2 g) and 48% aqueous hydrobromic acid (109.3 g) were added to the reaction mixture and this was heated to 95°C. The distillation apparatus was set to reflux return for the initial 30 minutes of reaction at 95°C. After this time, the toluene water azeotrope was collected in a separator with the toluene layer being recycled back to the reaction vessel. The reaction was heated for 5.5 hours at 95°C with toluene recycle. The mixture was cooled to 55°C with water (150 ml) and 48% aqueous hydrobromic acid (36.6 g) being added. This separation mixture was stirred at 55°C for 15 minutes and then allowed to settle for 30 minutes prior to the aqueous phase being removed. The remaining toluene/product layer was further washed with a pre-prepared mixture of water (150 ml) and 40% aqueous sodium acetate solution (36 g) this was stirred and settled prior to the aqueous buffer layer being discharged. The toluene layer was analysed for 2,3,5,6-tetrafluoro-4-(bromomethyl)benzyl alcohol product and gave a yield of 96.2%.

### Step e. Preparation of 4-methyl-2,3,5,6-tetrafluoro-benzyl alcohol

The following procedure used a reducer which was a 1 litre glass autoclave, working volume 350-500mls fitted with a gas dispersion agitator (1000rpm), gas feed through a dip-pipe via a Buchi gas controller type 6002, heating and cooling was controlled by a Jelabo FP40 bath. Methanol (362 g), water (6g), 2,3,5,6-tetrafluoro-4-(bromomethyl)benzyl alcohol (95.1 g 100% wt.), magnesium oxide (18.1 g) and 5% palladium/carbon catalyst, Johnson Matthey Type 58, (0.8 g 100% wt.) were charged to the autoclave. The lid was sealed and the autoclave was initially purged with nitrogen to remove residual oxygen and then pressurised to 2.5 bar g. with hydrogen after which the gas controller was zeroed. The agitator was started and the pressure maintained at 2.5 bar g. automatically by the Buchi controller. The reaction temperature was controlled at 50 °C throughout reaction by means of the external heating / cooling bath. The hydrogen consumption was totalled by the Buchi gas controller. The reaction was continued until the consumption of hydrogen ceased (typically 60 to 90 minutes). The residual pressure was released and the autoclave purged with nitrogen. The contents of the autoclave were discharged by the autoclave's bottom run-off valve, followed by a small methanol wash (30 g). The spent catalyst and inorganic salts was removed by filtration with the filter cake receiving methanol washes (2 x 30g). The filtrates and filter-cake washes were combined. On analysis the reaction had produced 60.4 g of 2,3,5,6-tetrafluoro-4-methylbenzyl alcohol, equating to a reaction yield of 89.4 %.

### EXAMPLE 3

This Example describes the preparation of 2,3,5,6-tetrafluoro-4-methylbenzyl *cis*-3-((Z)-2-chloro-3,3,3-trifluoroprop-1-enyl)-2,2-dimethylcyclopropane carboxylate Cis-Z 3-(2-chloro-1,1,1-trifluoro-2-propenyl)-2,2-dimethylcyclopropane carbonyl chloride (257 g) was charged to a reactor fitted with an agitator, thermometer, sub-surface nitrogen inlet and jacketed dropping funnel. Nitrogen sparging was applied to the acid chloride and maintained throughout subsequent processing. Molten 4-methyl-2,3,5,6-tetrafluorobenzyl alcohol (188.1 g) was charged to the dropping funnel. The temperature in the funnel was maintained at 80°C. The alcohol was charged to the acid chloride mixture over three hours. The maximum reaction temperature allowed during the addition was 45°C. On completion of the addition the temperature of the reaction mass was raised to 95°C and held for 2 hours. GC analysis was used to determine if full conversion of acid chloride had occurred and that no excess reactants were remaining. The mixture was cooled to 60°C and the molten reaction mass discharged, weighed and analysed. The reaction yielded 423.3 g of molten 2,3,5,6-tetrafluoro-4-methylbenzyl cis-3-((Z)-2-chloro-3,3,3-trifluoroprop-I-enyl)-2,2-dimethylcyclopropane carboxylate, analysis against a known standard showed this material to be 92.5 wt% giving an overall reaction yield of 96.5%.

## Claims

1. A process for the preparation of 4-methyl-2,3,5,6-tetrafluorobenzyl alcohol comprising:
a) fluorinating 2,3,5,6-tetrachloroterephthalonitrile to obtain 2,3,5,6-tetrafluoroterephthalonitrile;
b) hydrogenating 2,3,5,6-tetrafluoroterephthalonitrile to give 1,4-bis(aminomethyl)-2,3,5,6-tetrafluorobenzene;
c) converting 1,4-bis(aminomethyl)-2,3,5,6-tetrafluorobenzene to 1,4-bis(hydroxymethyl)-2,3,5,6-tetrafluorobenzene;
d) halogenation of 1,4-bis(hydroxymethyl)-2,3,5,6-tetrafluorobenzene to give a 4-(halomethyl)-2,3,5,6-tetrafluoro-benzyl alcohol where halo is chloro or bromo and
e) hydrogenation of the 4-(halomethyl)-2,3,5,6-tetrafluorobenzyl alcohol to give to 4-methyl-2,3,5,6-tetrafluorobenzyl alcohol.

2. A process according to claim 1 wherein step c) is performed by diazotisation of 1,4-bis(aminomethyl)-2,3,5,6-tetrafluorobenzene followed by *in-situ* hydrolytic decomposition of the resulting diazonium salt.

3. A process according to claim 1 or claim 2 which process comprises the further step of reacting 4-methyl-2,3,5,6-tetrafluorobenzyl alcohol with *cis*-Z 3-(2-chloro-1,1,1-trifluoro-2-propenyl)-2,2-dimethylcyclopropane carbonyl chloride to form tefluthrin.

4. A process according to any one of claims 1 to 3 in which the step of converting 1,4-bis(aminomethyl)-2,3,5,6-tetrafluorobenzene to 1,4-bis(hydroxymethyl)-2,3,5,6-tetrafluorobenzene is followed by a hydrolysis reaction to convert ester by-products to the required 1,4-bis(hydroxymethyl)-2,3,5,6-tetrafluorobenzene.

5. A process according to any one of claims 1 to 4 wherein the 2,3,5,6-tetrafluoroterephthalonitrile is hydrogenated in a carboxylic acid solvent and the hydrogenation mass is passed straight into the diazotisation stage (step c) after screening, such that no further acid is required.

6. A process according to any one of claims 1 to 5 wherein the reaction mass containing the 1,4-bis(hydroxymethyl)-2,3,5,6-tetrafluorobenzene is adjusted to pH 7-10 and the 1,4-bis(hydroxymethyl)-2,3,5,6-tetrafluorobenzene is extracted into a solvent.

7. A process according to any one of claims 1 to 6 wherein 1,4-bis(hydroxymethyl)-2,3,5,6-tetrafluorobenzene from step c) is extracted from the reaction mass in a solvent such as methyl isobutylketone (MiBK), the solvent removed under reduced pressure and a second solvent is added, the second solvent being suitable for the halogenation of 1,4-bis(hydroxymethyl)-2,3,5,6-tetrafluorobenzene to a 4-(halomethyl)-2,3,5,6-tetrafluoro-benzyl alcohol.

8. A process according to any one of claims 1 to 7 wherein the 1,4-bis(hydroxymethyl)-2,3,5,6-tetrafluorobenzene from step c) is extracted directly into a solvent that is also used in the halogenation of 1,4-bis(hydroxymethyl)-2,3,5,6-tetrafluorobenzene to a 4-(halomethyl)-2,3,5,6-tetrafluoro-benzyl alcohol.

## Patentansprüche

1. Verfahren zur Herstellung von 4-Methyl-2,3,5,6-tetrafluor-benzylalkohol, umfassend:
a) Fluorieren von 2,3,5,6-Tetrachlorterephthalnitril, um 2,3,5,6-Tetrafluorterephthalnitril zu erhalten;
b) Hydrieren von 2,3,5,6-Tetrafluorterephthalnitril, um 1,4-Bis(aminomethyl)-2,3,5,6-tetrafluorbenzol zu ergeben;
c) Umwandeln von 1,4-Bis(aminomethyl)-2,3,5,6-tetrafluorbenzol zu 1,4-Bis(hydroxymethyl)-2,3,5,6-tetrafluorbenzol;
d) Halogenierung von 1,4-Bis(hydroxymethyl)-2,3,5,6-tetrafluorbenzol, um einen 4-(Halogenmethyl)-2,3,5,6-tetrafluorbenzylalkohol zu ergeben, worin Halogen Chlor oder Brom darstellt, und
e) Hydrierung von dem 4-(Halogenmethyl)-2,3,5,6-tetrafluorbenzylalkohol, um 4-Methyl-2,3,5,6-tetrafluorbenzylalkohol zu ergeben.

2. Verfahren nach Anspruch 1, wobei Schritt c) durch Diazotierung von 1,4-Bis(aminomethyl)-2,3,5,6-tetrafluorbenzol, gefolgt von hydrolytischer *in-situ*-Zersetzung des erhaltenen Diazoniumsalzes, ausgeführt wird.

3. Verfahren nach Anspruch 1 oder Anspruch 2, wobei das Verfahren den weiteren Schritt des Umsetzens von 4-Methyl-2,3,5,6-tetrafluorbenzylalkohol mit *cis*-Z-3-(2-Chlor-1,1,1-trifluor-2-propenyl)-2,2-dimethylcyclopropancarbonylchlorid, unter Bildung von Tefluthrin, umfasst.

4. Verfahren nach einem der Ansprüche 1 bis 3, wobei dem Schritt des Umwandelns von 1, 4-Bis (aminomethyl)-2, 3, 5, 6-tetrafluorbenzol zu 1,4-Bis (hydroxymethyl)-2, 3, 5, 6-tetrafluorbenzol eine Hydrolysereaktion zum Umwandeln der Esternebenprodukte zu dem erforderlichen 1,4-Bis(hydroxymethyl)-2,3,5,6-tetrafluorbenzol folgt.

5. Verfahren nach einem der Ansprüche 1 bis 4, wobei das 2,3,5,6-Tetrafluorterephthalnitril in einem Carbonsäurelösungsmittel hydriert wird und die Hydrierungsmasse unmittelbar in die Diazotierungsstufe (Schritt c) nach Sieben (Screening) geleitet wird, sodass keine weitere Säure erforderlich ist.

6. Verfahren nach einem der Ansprüche 1 bis 5, wobei die Reaktionsmasse, die 1,4-Bis(hydroxymethyl)-2,3,5,6-tetrafluorbenzol enthält, auf pH 7-10 eingestellt wird und das 1,4-Bis(hydroxymethyl)-2,3,5,6-tetrafluorbenzol in ein Lösungsmittel extrahiert wird.

7. Verfahren nach einem der Ansprüche 1 bis 6, wobei 1,4-Bis(hydroxymethyl)-2,3,5,6-tetrafluorbenzol aus Schritt c) aus der Reaktionsmasse in ein Lösungsmittel, wie Methylisobutylketon (MiBK), extrahiert wird, wobei das Lösungsmittel unter vermindertem Druck entfernt wird, und ein zweites Lösungsmittel zugesetzt wird, wobei das zweite Lösungsmittel zur Halogenierung von 1,4-Bis(hydroxymethyl)-2,3,5,6-tetrafluorbenzol zu einem 4-(Halogenmethyl)-2,3,5,6-tetrafluorbenzylalkohol geeignet ist.

8. Verfahren nach einem der Ansprüche 1 bis 7, wobei 1,4-Bis(hydroxymethyl)-2,3,5,6-tetrafluorbenzol von Schritt c) direkt in ein Lösungsmittel, das auch bei der Halogenierung von 1,4-Bis(hydroxymethyl)-2,3,5,6-tetrafluorbenzol zu einem 4-(Halogenmethyl)-2,3,5,6-tetrafluorbenzylalkohol verwendet wird, extrahiert wird.

## Revendications

1. Procédé de préparation d'alcool 4-méthyl-2,3,5,6-tétrafluorobenzylique , comprenant:
a) la fluoration du 2,3,5,6-tétrachlorotéréphtalonitrile pour l'obtention de 2,3,5,6-tétrafluorotéréphtalonitrile;
b) l'hydrogénation du 2,3,5,6-tétrafluorotéréphtalonitrile pour l'obtention de 1,4-bis(aminométhyl)-2,3,5,6-tétrafluorobenzène;
c) la conversion du 1,4-bis(aminométhyl)-2,3,5,6-tétrafluorobenzène en le 1,4-bis(hydroxyméthyl)-2,3,5,6-tétrafluorobenzène;
d) l'halogénation du 1,4-bis(hydroxyméthyl)-2,3,5,6-tétrafluorobenzène pour l'obtention d'un alcool 4-(halogénométhyl)-2,3,5,6-tétrafluorobenzylique dans lequel halogéno est chloro ou bromo; et
e) l'hydrogénation de l'alcool 4-(halogénométhyl)-2,3,5,6-tétrafluorobenzylique pour l'obtention de l'alcool 4-méthyl-2,3,5,6-tétrafluorobenzylique.

2. Procédé selon la revendication 1, dans lequel l'étape c) s'effectue par diazotation du 1,4-bis(aminométhyl)-2,3,5,6-tétrafluorobenzène, suivie de la décomposition hydrolytique *in situ* du sel de diazonium résultant.

3. Procédé selon la revendication 1 ou la revendication 2, ce procédé comprenant l'étape supplémentaire de réaction de l'alcool 4-méthyl-2,3,5,6-tétrafluorobenzylique avec du chlorure de cis-Z-3-(2-chloro-1,1,1-trifluoro-2-propényl)-2,2-diméthylcyclopropanecarbonyle pour former de la téfluthrine.

4. Procédé selon l'une quelconque des revendications 1 à 3, dans lequel l'étape de conversion du 1,4-bis(aminométhyl)-2,3,5,6-tétrafluorobenzène en 1,4-bis(hydroxyméthyl)-2,3,5,6-tétrafluorobenzène est suivie d'une réaction d'hydrolyse pour convertir les sous-produits esters en le 1,4-bis(hydroxyméthyl)-2,3,5,6-tétrafluorobenzène désiré.

5. Procédé selon l'une quelconque des revendications 1 à 4, dans lequel le 2,3,5,6-tétrafluorotéréphtalonitrile est hydrogéné dans un solvant acide carboxylique et on fait passer directement le mélange d'hydrogénation dans l'étape de diazotation (étape c) après filtration, de sorte qu'il ne faut pas d'acide supplémentaire.

6. Procédé selon l'une quelconque des revendications 1 à 5, dans lequel on ajuste le mélange réactionnel contenant le 1,4-bis(hydroxyméthyl)-2,3,5,6-tétrafluorobenzène à pH 7-10 et on extrait le 1,4-bis(hydroxyméthyl)-2,3,5,6-tétrafluorobenzène dans un solvant.

7. Procédé selon l'une quelconque des revendications 1 à 6, dans lequel on extrait le 1,4-bis(hydroxyméthyl)-2,3,5,6-tétrafluorobenzène de l'étape c) dans un solvant comme la méthylisobutylcétone (MIBK), on sépare le solvant sous pression réduite et on ajoute un deuxième solvant, le deuxième solvant étant approprié pour l'halogénation du 1,4-bis(hydroxyméthyl)-2,3,5,6-tétrafluorobenzène en un alcool 4-(halogénométhyl)-2,3,5,6-tétrafluorobenzylique.

8. Procédé selon l'une quelconque des revendications 1 à 7, dans lequel on extrait le 1,4-bis(hydroxyméthyl)-2,3,5,6-tétrafluorobenzène de l'étape c) directement dans un solvant qui est aussi utilisé dans l'halogénation du 1,4-bis(hydroxyméthyl)-2,3,5,6-tétrafluorobenzène en un alcool 4-(halogénométhyl)-2,3,5,6-tétrafluorobenzylique.
